# NOUVEAU FASCICULE DE BREVET EUROPEEN

(11) **EP 1 889 606 B2**
(45) Date de publication et mention de la décision concernant l'opposition: **23.08.2017**
(45) Mention de la délivrance du brevet: 16.07.2014
(21) Numéro de dépôt: 07291097.9
(22) Date de dépôt: 22.11.2001
(51) Int. Cl.: A61K 8/81, A61Q 5/10, A61Q 5/06, A61K 8/87, A61K 8/73

(54) **Composition de teinture pour fibres kératiniques comprenant un polymère associatif anionique et un polymère à motifs acrylamide, halogenure de dialkyldiallylammonium et acide carboxylique vinylique**
Farbzusammensetzung für Keratinfasern bestehend aus einem anionischen assoziativen Polymer und einem Polymer mit Acrylamid-Einheiten sowie Dialkyldiallylammoniumhalogenid und Vinylcarbonsäure
Dyeing composition for keratinous fibres comprising an anionic associative polymer and a polymer with acrylamide, dialkyldiallylammonium halide and vinyl carboxylic acid patterns

(30) Priorité: 04.12.2000 FR 0015682
(43) Date de publication de la demande: 20.02.2008
(62) Demande divisionnaire de: 01999221.3
(73) Titulaire: L'Oréal, 75008 Paris (FR)
(72) Inventeur: Cottard, François, 92400 Courbevoie (FR); Rondeau, Christine, 78500 Sartrouville (FR)
(74) Mandataire: Wattremez, Catherine

(56) Documents cités:
- EP-A- 0 827 738
- EP-A- 0 897 711
- EP-A1- 0 827 739
- EP-A1- 0 966 948
- WO-A-94/10968
- WO-A-97/44002
- DE-A- 19 905 615
- FR-A- 2 773 992
- FR-A- 2 788 974

## Description

L'invention concerne une composition pour la teinture d'oxydation des fibres kératiniques, en particulier des fibres kératiniques humaines et plus particulièrement les cheveux, comprenant, dans un milieu approprié pour la teinture, au moins un colorant d'oxydation et au moins un polymère associatif anionique et qui est caractérisée par le fait qu'elle comprend en outre au moins un polymère à motifs (i) acrylamide, (ii)halogénure de dialkyldiallylammonium, et (iii) acide carboxylique vinylique.
L'invention concerne également les procédés et dispositifs de teinture mettant en oeuvre ladite composition de teinture.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration directe éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.

Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
La variété des molécules mises en jeu, qui sont constituées d'une part, par les bases d'oxydation et d'autre part, par les coupleurs, permet l'obtention d'une palette très riche en coloris.
Pour varier encore les nuances obtenues avec lesdits colorants d'oxydation, ou les enrichir de reflets, Il arrive qu'on leur ajoute des colorants directs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour localiser le produit de coloration d'oxydation à l'application sur les cheveux afin qu'il ne coule pas sur le visage ou en dehors des zones que l'on se propose de teindre, outre l'emploi d'épaississants traditionnels tels que l'acide polyacrylique réticulé, les hydroxyéthylcelluloses, les cires, et des mélanges d'agents tensio-actifs non-ioniques de HLB (Hydrophilic Lipophilic Balance), convenablement choisis, on a aussi eu recours à des polymères associatifs de type anionique, non-ionique ou cationique.
Les polymères associatifs de type anionique, non-ionique ou cationique représentent un progrès important dans la recherche d'une solution à ce problème.

Voici maintenant que la demanderesse a découvert, de façon tout à fait inattendue et surprenante, de nouvelles compositions de teinture, optimales quant à l'application sur les fibres (ne coulent pas et restent donc mieux localisées au point d'application), quant à obtenir des nuances puissantes et chromatiques avec de faibles sélectivités et de bonnes ténacités, quant aux propriétés cosmétiques conférées à la chevelure traitée, lesdites compositions de teinture comprenant dans la composition colorante, ou dans la composition oxydante (lorsqu'il s'agit de teinture d'oxydation), ou dans les deux compositions à la fois, outre au moins un polymère associatif anionique particulier, une quantité efficace d'au moins un polymère à motifs (i)acrylamide, (ii)halogénure de dialkyldiallylammonium, et (iii) acide carboxylique vinylique.
Cette découverte est à la base de la présente invention.

La présente invention a ainsi pour objet une nouvelle composition pour la teinture des fibres kératiniques, en particulier des fibres kératiniques humaines, telles que les cheveux, comprenant, dans un milieu approprié pour la teinture, au moins un colorant d'oxydation et au moins un polymère associatif anionique particulier et qui est caractérisée par le fait qu'elle contient en outre au moins un polymère à motifs (i) acrylamide, (ii)hatogénure de dialkyldiallylammonium, et (iii) acide carboxylique vinylique.

Un autre objet de la présente invention porte sur une composition prête à l'emploi pour la teinture des fibres kératiniques, en particulier des fibres kératiniques humaines, telles que les cheveux comprenant au moins un colorant d'oxydation au moins un polymère associatif anionique particulier, au moins un polymère à motifs (i) acrylamide, (ii)halogénure de dialkyldiallylammonium, et (iii) acide carboxylique vinylique, et en outre au moins un agent oxydant

Au sens de la présente invention, on entend par composition prête à l'emploi, toute composition destinée à être appliquée immédiatement sur les fibres kératiniques; elle peut donc être stockée telle quelle avant utilisation ou résulter du mélange extemporané de deux ou plusieurs compositions.

L'invention vise également un procédé de teinture des fibres kératiniques, et en particulier des fibres kératiniques humaines telles que les cheveux, consistant à appliquer sur les fibres au moins une composition colorante comprenant, dans un milieu approprié pour la teinture, au moins un colorant (direct ou d'oxydation), la couleur étant révélée à pH alcalin, neutre ou acide, à l'aide d'une composition oxydante contenant au moins un agent oxydant qui est mélangée juste au moment de l'emploi à la composition colorante
ou qui est appliquée séquentiellement sans rinçage intermédiaire, au moins un polymère associatif anionique particulier et au moins un polymère à motifs (i) acrylamide, (ii)halogénure de dialkyldiallylammonium, et (iii) acide carboxylique vinylique étant présents dans la composition colorante ou dans la composition oxydante ou dans chacune desdites compositions.

Dans un procédé préféré, les polymères associatif(s) et les polymères à motifs (i) acrylamide, (ii)halogénure de dialkyldiallylammonium, et (iii) acide carboxylique vinylique sont présents dans la composition colorante.

L'invention a également pour objet des dispositifs de teinture ou « kits » à plusieurs compartiments.

Un dispositif à 2 compartiments selon l'invention comprend un compartiment renfermant une composition colorante comprenant, dans un milieu approprié pour la teinture, au moins un colorant (direct ou d'oxydation), et un autre compartiment renfermant une composition oxydante comprenant, dans un milieu approprié pour la teinture, un agent oxydant, au moins un polymère associatif anionique et au moins un polymère à motifs (i) acrylamide, (ii)halogénure de dialkyldiallylammonium, et (iii) acide carboxylique vinylique étant présents dans la composition colorante ou la composition oxydante, ou dans chacune de ces compositions.

Mais d'autres caractéristiques, aspects, objets et avantages de l'invention apparaîtront encore plus clairement à la lecture de la description et des exemples qui suivent.

### Polymères à motifs (i) acrylamide, (ii)halogénure de dialkyldiallylammonium, et (iii) acide carboxylique vinylique, utilisés selon l'invention.

Selon l'invention, on entend désigner par motifs acrylamide des motifs de structure (I): 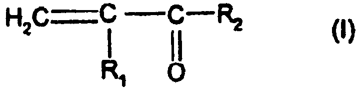 dans laquelle,
- R₁ désigne H ou CH₃,
- R₂ est choisi parmi un radical amino, diméthylamino, tert-butylamino, dodécylamino, ou -NH-CH₂OH.
Le motif acrylamide [formule (I) dans laquelle R₁ est H et R₂ est amino] est particulièrement préféré.

Les motifs halogénure de dialkyldiallylammonium sont de structure (II) suivante: 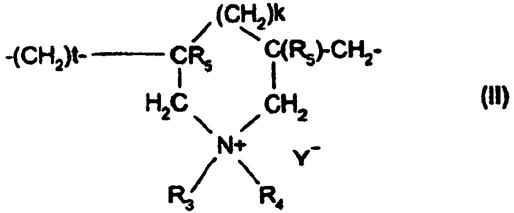 formule dans laquelle k et t sont égaux à 0 ou 1, la somme k + t étant égale à 1 ; R₅ désigne un atome d'hydrogène ou un radical méthyle ; R₃ et R₄, indépendamment l'un de l'autre, désignent un groupement alkyle ayant de 1 à 4 atomes de carbone, un groupement hydroxyalkyle dans lequel le groupement alkyle a de préférence 1 à 5 atomes de carbone, un groupement amidoalkyle(C₁-C₄), ou R₃ et R₄ peuvent désigner conjointement avec l'atome d'azote auquel ils sont rattachés, des groupements hétérocycliques, tels que pipéridinyle ou morpholinyle ; R₃ et R₄ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate.
Parmi ces motifs halogénure de dialkyldiallylammonium, on préfère le chlorure de diméthyldiallylammonium.

Selon l'invention, on entend également désigner par motifs acide carboxylique vinylique, l'acide acrylique, l'acide méthacrylique et l'acide 2-acrylamido-2-méthyl-propane sulfonique.
Le motif acide acrylique est particulièrement préféré.

Parmi ces polymères à motifs (i) acrylamide, (ii) halogénure de dialkyldiallylammonium, et (iii) acide carboxylique vinylique, on peut citer notamment les terpolymères acrylamide / chlorure de diméthyldiallylammonium / acide acrylique, répertoriés dans le dictionnaire C.T.F.A. International Cosmetic Ingredient Dictionary, 7ème édition 1997, sous la dénomination "POLYQUATERNIUM 39" ; des exemples de tels polymères ont les compositions pondérales suivantes :
- (25/50/25), proposé par la société CALGON,
   sous l'appellation MERQUAT PLUS 3330 DRY®,
   en solution aqueuse à 10% sous l'appellation MERQUAT PLUS 3330®,
   en solution aqueuse à 23,6% sous l'appellation POLYMER 3413-54,
   en solution aqueuse à 23,3% sous l'appellation POLYMER 3413-58,
   en solution aqueuse à 21,3% sous l'appellation POLYMER 3413-62,
- (50/40/10), proposé par la société CALGON,
   en solution aqueuse à 5% sous l'appellation POLYMER 3120-59,
- (50/35/15), proposé par la société CALGON,
   en solution aqueuse à 5% sous l'appellation POLYMER 3120-61,
- (50/30/20), proposé par la société CALGON,
   en solution aqueuse à 4,6% sous l'appellation POLYMER 3120-63; on peut également citer le terpolymère acrylamide / chlorure de diméthyldiallylammonium / acide 2-acrylamido-2-méthyl-propane sulfonique proposé en solution aqueuse à 8% par la société CALGON sous l'appellation POLYMER 3575-53.

Selon l'invention, le ou les polymères à motifs (i)acrylamide, (ii)halogénure de dialkyldiallylammonium, et (iii)acide carboxylique vinylique représentent environ 0,1% à 10% en poids, de préférence environ 0,5% à 5% en poids, et plus particulièrement environ 1 % à 3% en poids du poids total de la composition de teinture.

### Polymères associatifs utilisés selon l'invention

Les polymères associatifs sont des polymères hydrosolubles, capables dans un milieu aqueux, de s'associer réversiblement entre eux ou avec d'autres molécules.
Leur structure chimique comprend des zones hydrophiles, et des zones hydrophobes caractérisées par au moins une chaîne grasse.
Les polymères associatifs selon l'invention sont de type anionique.

Les polymères associatifs de type anionique sont choisis parmi les polymères suivants :
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé, choisi parmi l'acide acrylique, l'acide méthacrylique et l'acide éthacrylique.
   De préférence, ces polymères sont choisis parmi ceux dont le motif hydrophile de type acide carboxylique insaturé oléfinique correspond au monomère de formule (II) suivante : 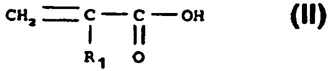 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante : 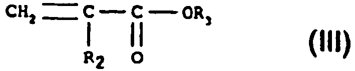 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères associatifs anioniques, on utilisera plus particulièrement des polymères formés à partir d'un mélange de monomères comprenant :
   (i) essentiellement de l'acide acrylique,
   (ii) un ester de formule (III) décrite ci-dessus et dans laquelle R₂ désigne H ou CH₃, R₃ désignant un radical alkyle ayant de 12 à 22 atomes de carbone,
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
      Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précedemment.
      Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.
- (V) les copolymères comportant parmi leurs monomères un acide carboxylique à insaturation α,β-monoéthylénique et un ester d'acide carboxylique à insaturation α ,β-monoéthylénique et d'un alcool gras oxyalkyléné correspondant à un terpolymère acide méthacrylique/acrylate d'éthyle/méthacrylate de stéaryle oxyalkyléné. A titre d'exemple de ce type de composé on peut citer l'ACULYN 22 vendu par la société ROHM et HAAS.

Les polymères associatifs anioniques sont utilisés de préférence en une quantité pouvant varier d'environ 0,1 à 10% en poids du poids total de la composition de teinture. Plus préférentiellement, cette quantité varie d'environ 0,5 à 5% en poids, et encore plus particulièrement d'environ 1 à 3% en poids.

### Colorants d'oxydation

Les colorants d'oxydation utilisables selon l'invention sont choisis parmi les bases d'oxydation et/ou les coupleurs.
De préférence les compositions selon l'invention contiennent au moins une base d'oxydation.

La nature de ces bases d'oxydation n'est pas critique. Elles peuvent notamment être choisies parmi les ortho- et para-phénylènediamines, les bases doubles, les ortho- et para- aminophénols, les bases hétérocycliques suivantes ainsi que les sels d'addition de tous ces composés avec un acide.

On peut notamment citer :
- (I) les paraphénylènediamines de formule (I) suivante et leurs sels d'addition avec un acide : 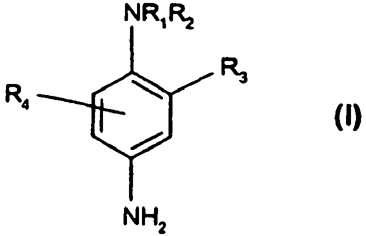 dans laquelle:
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylénediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylénediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (II) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 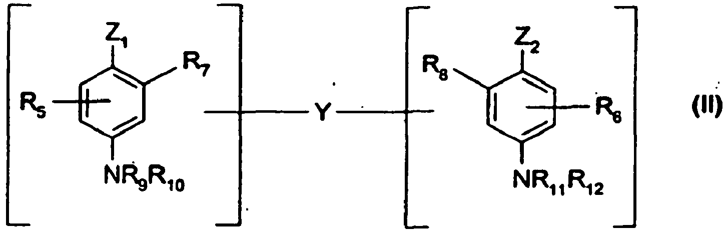 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diaminopropanol, le 1,8-bis-(2,5-diaminophénoxy)-3,5-dioxaoctane ou l'un de leurs sels d'addition avec un acide sont particulièrement préférés.
- (III) les para-aminophénols répondant à la formule (III) suivante, et leurs sels d'addition avec un acide: 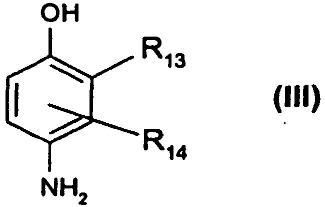 dans laquelle :
   R₁₃ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (IV) les ortho-aminophénols utilisables à titre de bases d'oxydation dans le cadre de la présente invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine : la pyrazolo-[1,5-a]-pyrimidine-3,5-diamine ; la 2,7-diméthyl-pyrazolo-[1,5-a]-pyrimidine-3,5-diamine le 3-amino-pyrazolo-[1,5-a]-pyrimidin-7-ol ; le 3-amino-pyrazo-lo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(1-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94108969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

La composition tinctoriale conforme à invention peut renfermer un ou plusieurs coupleurs choisis parmi ceux classiquement utilisés en teinture d'oxydation et notamment parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition tinctoriale et encore plus préférentiellement de 0,001 à 10% environ.

Les sels d'addition avec un acide de ces colorants d'oxydation (bases et/ou coupleurs) sont notamment choisis parmi les chlorhydrates, les bromhydrates, les sulfates et les tartrates, les lactates et les acétates.

La composition tinctoriale conforme à invention comprenant au moins un colorant d'oxydation peut en outre renfermer un ou plusieurs colorants directs notamment pour modifier les nuances obtenues avec les bases et/ou les coupleurs en les enrichissant de reflets. Ces colorants directs peuvent notamment être choisis parmi les colorants nitrés, azoïques ou anthraquinoniques, neutres, cationiques ou anioniques, classiquement utilisés ou ceux décrits notamment dans les demandes de brevet FR-2782450, 2782451, 2782452 et EP-1025834, dans la proportion pondérale d'environ 0,001 à 20% et de préférence de 0,01 à 10% du poids total de la composition.

### Colorants directs

Les colorants directs convenant pour la teinture directe sont ceux décrits ci-dessus et ceux convenant plus particulièrement pour la teinture éclaircissante (c'est à dire avec un oxydant) sont ceux décrits notamment dans les demandes de brevet FR-2782450, 2782451, 2782452 et EP-1025834.

### Milieu

Le milieu de la composition approprié pour la teinture, est de préférence un milieu aqueux constitué par de l'eau pouvant comprendre avantageusement des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propyléneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% et, de préférence, entre environ 2 et 10% en poids par rapport au poids total de la composition.

La composition tinctoriale et/ou la composition oxydante (dans le cas d'une teinture d'oxydation ou d'une teinture éclaircissante) peuvent en outre plus particulièrement comprendre, au moins un agent tensio-actif anionique, non ionique, cationique ou amphotère ou zwittérionique dans la proportion d'au moins 0,01 % en poids par rapport au poids total de la composition, et de préférence un agent tensio-actif de nature non-ionique.

Ces agents tensio-actifs peuvent être choisis parmi :

### Les tensioactifs non ioniques :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylamino-propylmorpholine.

### Les tensioactifs anioniques :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### Les tensioactifs amphotères ou zwittérioniques:

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂ désigne un radical alkyle d'un acide Rg -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylampho-dipropionate, Disodium Capryloamphodipropionate, Lauroamphodipropionic acid, Coco-amphodipropionic acid.
A titre d'exemple on peut citer le cocoamphodiacetate commercialisé sous la dénomination commerciale MIRANOL^{®} C2M concentré par la société RHODIA CHIMIE.

### Les tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition de teinture selon l'invention peuvent varier de 0,01 à 40% et de préférence de 0,5 à 30% du poids total de la composition.

De préférence, selon l'invention, la composition colorante et/ou la composition oxydante peuvent en outre plus particulièrement comprendre, au moins un polymère cationique ou amphotère (différent des polymères associatifs et des polymères à motifs (i) acrylamide, (ii)halogénure de dialkyldiallylammonium, et (iii) acide acrylique, selon l'invention) dans la proportion d'au moins 0,01% en poids par rapport au poids total de la composition.
Plus particulièrement selon l'invention, les dits polymères cationiques ou amphotères sont dans la partie colorante.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 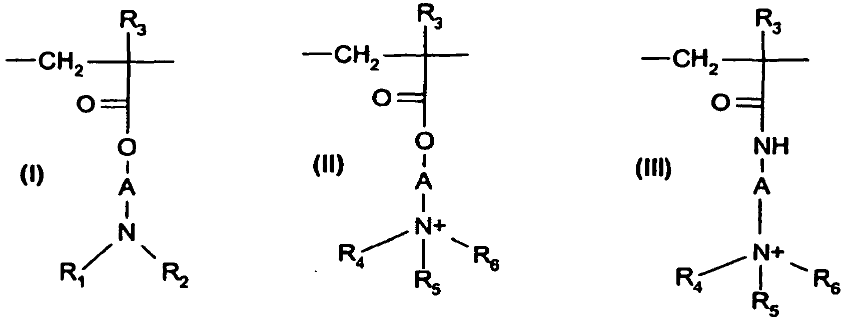 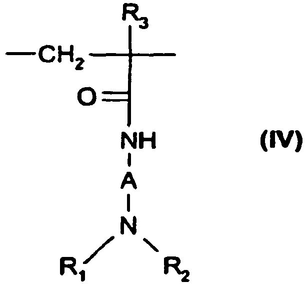 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

### Les polymères de la famille

(1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC® par la société HERCULES,
   - les copolymères d'acrylamide et de chlorure de méthacryloyloxyéthyltriméthyl-ammonium décrits par exemple dans la demande de brevet EP-A-080976 et vendus sous la dénomination BINA QUAT P 100® par la société CIBA GEIGY,
   - le copolymère d'acrylamide et de méthosulfate de méthacryloyloxyéthyltriméthyl-ammonium vendu sous la dénomination RETEN® par la société HERCULES,
   - les copolymères vinylpyrrolidone / acrylate ou méthacrylate de dialkylaminoalkyle quatemisés ou non, tels que les produits vendus sous la dénomination "GAFQUAT®" par la société ISP comme par exemple "GAFQUAT 734" ou "GAFQUAT 755" ou bien les produits dénommés "COPOLYMER 845®, 958® et 937®". Ces polymères sont décrits en détail dans les brevets français 2.077.143 et 2.393.573,
   - les terpolymères méthacrylate de diméthyl amino éthyle/ vinylcaprolactame/ vinylpyrrolidone tel que le produit vendu sous la dénomination GAFFIX VC 713® par la société ISP,
   - les copolymère vinylpyrrolidone / méthacrylamidopropyl dimethylamine commercialisés notamment sous la dénomination STYLEZE CC 10® par ISP,
   - et les copolymères vinylpyrrolidone / méthacrylamide de diméthylaminopropyle quatemisés tel que le produit vendu sous la dénomination "GAFQUAT HS 100®" par la société ISP.
(2) Les dérivés d'éthers de cellulose comportant des groupements ammonium quaternaire décrits dans le brevet français 1 492 597, et en particulier les polymères commercialisés sous les dénominations "JR®" (JR 400, JR 125, JR 30M) ou "LR®" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200®" et "Celquat H 100®" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium. De tels produits sont commercialisés notamment sous les dénominations commerciales de JAGUAR C13 S®, JAGUAR C 15®. JAGUAR C 17® ou JAGUAR C162® par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultantde la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quatemisées. De tels polymères sont notamment décrits dans les brevets français 2.252.840 et 2.368.508.
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine® F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57®" par la société Hercules Inc. ou bien sous la dénomination de "PD 170®" ou "Delsette 101®" par la société Hercules dans le cas du copolymère d'acide adipique/époxypropyl/diéthylène-triamine.
(9) Les cyclopolymères de dialkyl diallyl ammonium tels que les homopolymères ou copolymères comportant comme constituant principal de la chaîne des motifs répondant à la formule (V) : 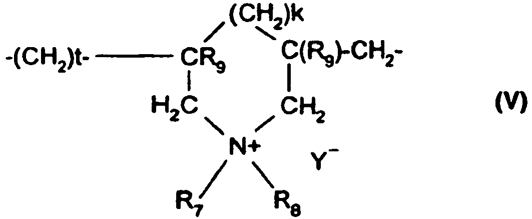
   dans laquelle k et t sont égaux à 0 ou 1, la somme k + t étant égale à 1 ;
   R₇ et R₈, identiques ou différents, désignent un groupement alkyle ayant de 1 à 8 atomes de carbone, un groupement hydroxyalkyle(C₁-C₅), un groupement amidoalkyle(C₁-C₄), ou R₇ et R₈ désignent conjointement avec l'atome d'azote auquel ils sont rattachés, un groupement pipéridinyle ou morpholinyle ;
   R₉ désigne un atome d'hydrogène ou un radical méthyle ;
   R₇ et R₈ indépendamment l'un de l'autre désignent de préférence un groupement alkyle ayant de 1 à 4 atomes de carbone ; Y est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100®" par la société Calgon (et ses homologues de faible masse moléculaire moyenne en poids) et les copolymères de chlorure de diallyldiméthylammonium et d'acrylamide commercialisés sous la dénomination "MERQUAT 550®".
(10) Le polymère de diammonium quaternaire contenant des motifs récurrents répondant à la formule : 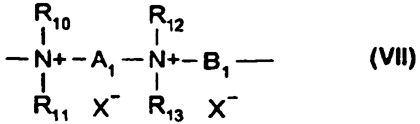 formule (VII) dans laquelle :
   R₁₀, R₁₁, R₁₂ et R₁₃, identiques ou différents, représentent des radicaux aliphatiques, anticycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire :
   A1 et B1 représentent des groupements polyméthyléniques contenant de 2 à 20 atomes de carbone pouvant être linéaires ou ramifiés, saturés ou insaturés, et pouvant contenir,
   liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester,
      et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (VIII) suivante: 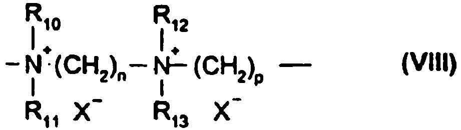 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(11) Les polymères de polyammonium quaternaire constitués de motifs récurrents de formule (IX) : 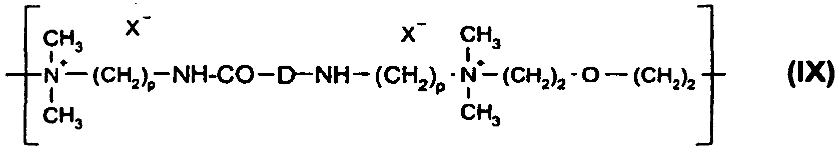 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15®", "Mirapol AD1®", "Mirapol AZ1®" et "Mirapol 175®" vendus par la société Miranol.
(12) Les polymères quaternaires de vinylpyrrolidone et de vinylimidazole tels que par exemple les produits commercialisés sous les dénominations Luviquat FC 905®, FC 550® et FC 370® par la société B.A.S.F.
(13) Les polyamines comme le Polyquart H® vendu par HENKEL, référencé sous le nom de " POLYETHYLENEGLYCOL (15) TALLOW POLYAMINE " dans le dictionnaire CTFA.
(14) Les polymères réticulés de sels de méthacrytoyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (9), (10) et (11) et encore plus préférentiellement les suivants :
- 1/ l'homopolymère de chlorure de diméthyldiallylammonium (famille 9);
- 2/ les polymères aux motifs récurrents de formule (VIII) décrite ci-avant (famille 10) et pour laquelle R₁₀, R₁₁, R₁₂ et R₁₃ représentent un radical méthyle, n = 3, p = 6 et X = Cl, et notamment ceux dont le poids moléculaire, déterminé par chromatographie par perméation de gel, est compris entre 9500 et 9900 [Polymère W de formule ci-dessous];
   R₁₀ et R₁₁ représentent un radical méthyle, R₁₂ et R₁₃ représentent un radical éthyle et n = p = 3 et X = Br, et notamment ceux dont le poids moléculaire, déterminé par chromatographie par perméation de gel, est d'environ 1200 [Polymère U de formule ci-dessous]. 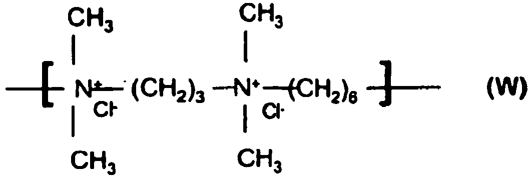 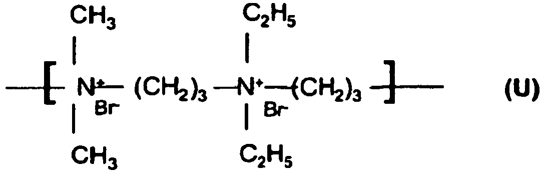
   Lesdits polymères à motifs (W) et (U) sont préparés et décrits dans le brevet français 2 270 846.
- 3/ les polymères à motifs de formule (IX) décrite ci-dessus (famille 11), pour laquelle, p est égal à 3, et,
   a) D désigne la valeur zéro, X désigne un atome de chlore, la masse moléculaire mesurée par RMN du Carbone 13, (RMN¹³C) étant d'environ 25500 ; un polymère de ce type est vendu par la société MIRANOL sous le nom MIRAPOL-A15®,
   b) D représente un groupement -(CH₂)₄-CO-, X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-AD1®.
   c) D représente un groupement -(CH₂)₇-CO-, X désigne un atome de chlore, la masse moléculaire mesurée par RMN du Carbone 13 (RMN¹³C) étant d'environ 8100; un polymère de ce type est proposé par la société MIRANOL sous le nom de MIRAPOL-AZ1®,
   d) un " Block Copolymer " formé de motifs correspondant aux polymères décrits aux alinéas a) et b), proposé par la société MIRANOL sous les noms MIRAPOL-9®, (masse moléculaire RMN¹³C, environ 7800) MIRAPOL-175®, (masse moléculaire RMN¹³C, environ 8000) MIRAPOL-95®, (masse moléculaire RMN¹³C, environ 12500). Plus particulièrement encore, on préfère selon l'invention, le polymère à motifs de formule (IX) dans laquelle p est égal à 3, D désigne la valeur zéro, X désigne un atome de chlore, la masse moléculaire mesurée par RMN du Carbone 13 (RMN¹³C) étant d'environ 25500 (Mirapol-A15®).

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033® par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280® et MERQUAT 295®, par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique
   et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-terdobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
   On utilise particulièrement les copolymères dont la dénomination CTFA (4ème Ed., 1991) estOctylacrylamide/acrylates/butylaminoethylmethacrylate copolymer tels que les produits vendus sous la dénomination AMPHOMER® ou LOVOCRYL 47® par la société NATIONAL STARCH.
(3) Les polyaminoamides réticulés et alcoylés partiellement ou totalement dérivant de polyaminoamides de formule générale:

   -[CO-R₁₉-CO-Z-] (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 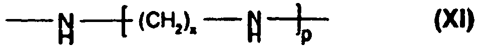 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 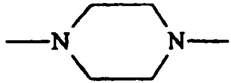
   c) dans les proportions de 0 à 20 moles % le radical -NH-(CH₂)₆-NH- dérivant de l'hexaméthylénediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 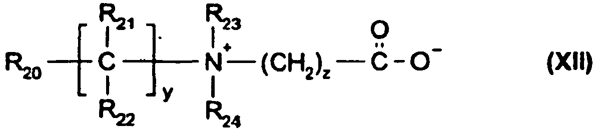 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301® par la société SANDOZ.
(5) les polymères dérivés du chitosane décrits notamment dans les brevets français N°-2137684 ou US-3879376, comportant des motifs monomères répondant aux formules (XIII). (XIV), (XV) suivantes réunies dans leur chaîne: 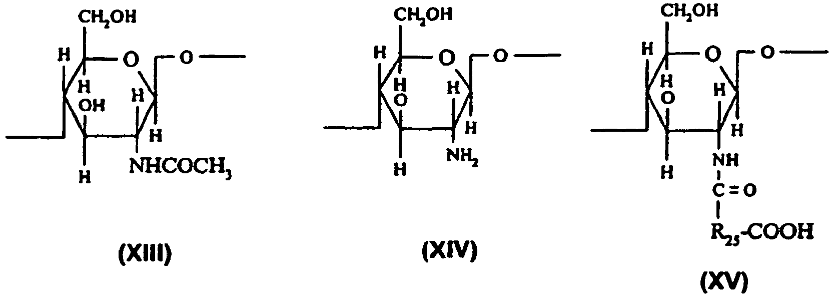 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formula : 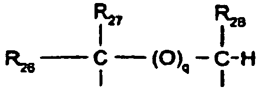 dans laquelle q désigne zéro ou 1 ;
   si q=0. R₂₆. R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompues par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
   Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical -CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN®" par la société JAN DEKKER.
(7) Les polymères répondant à la formule générale (XI) tels que ceux décrits par exemple dans le brevet français 1 400 366 : 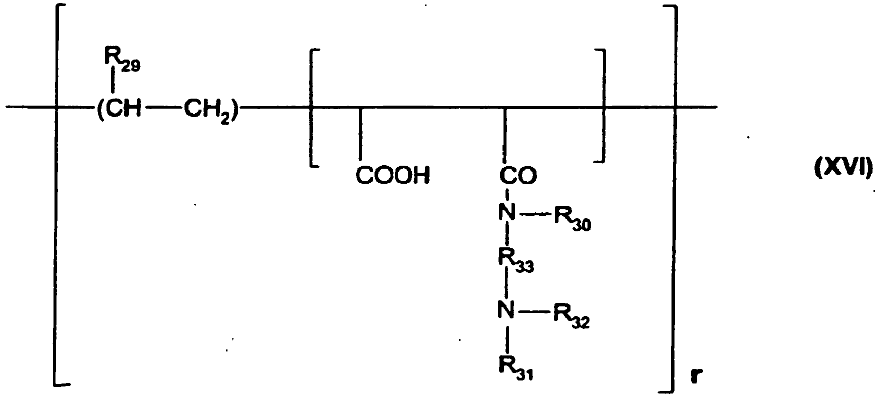 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 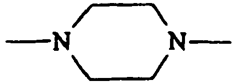 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)

      où D désigne un radical 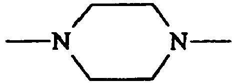 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminaalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères amphotères susceptibles d'être utilisés dans le cadre de la présente invention, on préfère mettre en oeuvre les polymères de la famille (1), et en particulier les copolymères d'acide acrylique et de chlorure de diméthyldiallylammonium.

Selon l'invention, le ou les polymères cationiques ou amphotères différant des polymères associatifs cationiques ou amphotères et des polymères à motifs (i) acrylamide, (ii)halogénure de dialkyldiallylammonium, et (iii) acide acrylique selon l'invention, peuvent représenter environ 0,01 % à 10 % en poids, de préférence 0,05 % à 5 % en poids, et encore plus préférentiellement 0,1 % à 3 % en poids, du poids total de la composition.

La composition colorante peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....
La composition colorante peut également comprendre d'autres agents d'ajustement de la rhéologie tels que les épaississants cellulosiques (hydroxyéthycellulose, hydroxypropylcellulose, carboxyméthylcellulose..), la gomme de guar et ses dérivés(hydroxypropylguar..), les gommes d'origine microbienne (gomme de xanthane, gomme de scléroglucane..), les épaississants synthétiques tels que les homopolymères réticulés d'acide acrylique ou d'acide acrylamidopropane-sulfonique.
Ces épaississants d'appoint peuvent représenter de 0,01 à 10% en poids du poids total de la composition.

Ladite composition peut également comprendre des agents réducteurs ou antioxydants. Ceux-ci peuvent être choisis en particulier parmi le métabisulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition oxydante, l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les oxydoréductases à 4 électrons (comme les laccases), les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale et de la composition oxydante], est généralement compris entre les valeurs 3 et 12. Il est de préférence compris entre 8,5 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 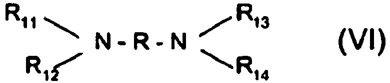 dans laquelle R est un reste propylène éventuellement substitué par un groupement hydroxyle ou un radical alkyle en C₁-C₄ ; R₁₁, R₁₂, R₁₃ et R₁₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer un mélange, réalisé extemporanément au moment de l'emploi, (composition prête à l'emploi) à partir des compositions colorante et oxydante décrites ci-avant, sur les fibres kératiniques sèches ou humides, et à le laisser agir pendant un temps de pause variant, de préférence, de 1 à 60 minutes environ, et plus préférentiellement de 5 à 45 minutes environ, à rincer les fibres, puis éventuellement à les laver au shampooing, puis à les rincer à nouveau, et à les sécher.

Des exemples concrets illustrant l'invention vont maintenant être donnés, sans pour autant présenter un caractère limitatif.

### EXEMPLE 1 :

On a préparé la composition de teinture d'oxydation, non-conforme à l'nvention, suivante
(exprimée en grammes)
M.A* désigne Matière Active

**Composition colorante :**

| | |
|---|---|
| Mélange d'alcools linéaires en C18 à C24 [C18/C20/C22/C24: 7/58/30/6 -teneur en alcool > 95%] | 3 |
| Mélange d'alcools linéaires en C18 à C24 [C18/C20/C22/C24 : 7/58/30/6 -teneur en alcool > 95%] oxyéthylénés (30 OE | 1 |
| Alcool stéarylique oxyéthyléné (2 OE) | 4,5 |
| Alcool stéarylique oxyéthyléné (21 OE) | 1.75 |
| Acide oléique | 2,6 |
| Acide polyacrylique réticulé (Carbopol 980 de GOODRICH) | 0.6 |
| Aculyn 44 ou Aculyn 46 vendu par ROHM & HAAS | 4 |
| Monoisopropanolamide d'acides de coprah | 3 |
| MERQUAT PLUS 3330 vendu par CALGON en solution aqueuse à 10% MA | 1,2 MA* |
| Propylène glycol | 6 |
| Métabisulfite de sodium | 0.71 |
| EDTA (Acide éthylènediamine tétra-acétique | 0.2 |
| Ter-butyl hydroquinone | 0.3 |
| 1,4-diammobenzène | 0.5 |
| Para-aminophénol | 0,1 |
| 1,3-dihydroxybenzène | 0.6 |
| 1-hydroxy-3-amino-benzene | 0,1 |
| 1-(3-hydroxyethyloxy-2.4-diammo-benzène, dichlorhydrate | 0,04 |
| Monoéthanolamine | 1 |
| Ammoniaque à 20% de NH3 | 11 |
| Parfum q.s | |
| Eau déminéralisée q.s.p | 100 |

**Composition oxydante :**

| | |
|---|---|
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée | qsp 100 |

Ladite composition colorante a été mélangée, au moment de l'emploi, dans un bol en plastique à la composition oxydante donnée ci-dessus, à raison de 1 partie de composition colorante pour 1,5 parties de composition oxydante.
On a appliqué le mélange obtenu sur des mèches de cheveux naturels à 90% de blancs et on a laissé poser 30 minutes. On a ensuite rincé les mèches à l'eau, on les a lavées au shampooing standard, à nouveau rincées à l'eau, puis séchées et démélées.
Les cheveux ont été teints dans une nuance blond foncé, tenace, et l'état cosmétique du cheveu a été amélioré.

### EXEMPLE 2 :

On a reproduit la composition de l'exemple 1, en remplaçant les 4 grammes d'Aculyn 44® ou d'Aculyn 46® par 3.8g MA de Pemulen TR1® vendu par GOODRICH. En suivant le même protocole qu'à l'exemple 1, la composition de teinture d'oxydation obtenue a donné des performances identiques en terme d'état cosmétique du cheveu et de puissance de la teinture.

## Revendications

1. Composition pour la teinture des fibres kératiniques, en particulier des fibres kératiniques humaines, et plus particulièrement les cheveux, comprenant, dans un milieu approprié pour la teinture, au moins un colorant d'oxydation et au moins un polymère associatif anionique et qui est **caractérisée par le fait qu'**elle contient en outre au moins un polymère à motifs (i) acrylamide, (ii) halogénure de dialkyldiallylammonium, et (iii) acide carboxylique vinylique ; ledit polymère associatif anionique étant choisi parmi les polymères suivants :
- (II) ceux comportant au moins un motif hydrophile de type acide carboxylique insaturé oléfinique et au moins un motif hydrophobe de type ester d'alkyl (C₁₀-C₃₀) d'acide carboxylique insaturé, choisi parmi l'acide acrylique, l'acide méthacrylique et l'acide éthacrylique,
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

2. Composition selon la revendication 1, **caractérisée par le fait que** le ou les colorants d'oxydation sont choisis parmi les bases d'oxydation et/ou les coupleurs.

3. Composition selon la revendication 2, **caractérisée par le fait que** les bases d'oxydation sont choisies parmi les ortho- ou para-phénylènediamines, les bases doubles, les ortho- ou para-aminophénols, et les bases hétérocycliques, ainsi que les sels d'addition de ces composés avec un acide, et sont présentes dans des concentrations allant de 0,0005 à 12% en poids par rapport au poids total de la composition.

4. Composition selon la revendication 2, **caractérisée par le fait que** les coupleurs sont choisis parmi les métaaminophénols, les métaphénylènediamines, les métadiphénols, les naphtols, les coupleurs hétérocycliques, et les sels d'addition de ces composés avec un acide, et sont présents dans des concentrations allant de 0,0001 à 10% en poids par rapport au poids total de la composition.

5. Composition selon l'une quelconque des revendications 3 ou 4, **caractérisée par le fait que** les sels d'addition avec un acide des colorants d'oxydation (bases et coupleurs) sont choisis parmi les chlorhydrates, les bromhydrates, les sulfates, les tartrates, les lactates et les acétates.

6. Composition selon la revendication 2, **caractérisée par le fait qu'**elle renferme en outre au moins un agent oxydant.

7. Composition selon la revendication 6, **caractérisée par le fait qu'**elle contient en outre au moins un colorant direct dans la proportion de 0,001 à 20% en poids par rapport au poids total de la composition.

8. Composition selon l'une quelconque des revendications précédentes, **caractérisée par le fait que** dans le polymère à motifs (i) acrylamide, (ii) halogénure de dialkyldiallylammonium, et (iii) acide carboxylique vinylique, le motif acrylamide est choisi parmi les motifs de structure (I) suivante: 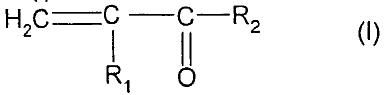 dans laquelle,
- R₁ désigne H ou CH₃,
- R₂ est choisi parmi un radical amino, diméthylamino, tert-butylamino, dodécylamino, ou -NH-CH₂OH.

9. Composition selon la revendication 9, **caractérisée par** le fait dans la formule (I) R₁ est hydrogène et R₂ est un radical amino.

10. Composition selon l'une quelconque des revendications précédentes, **caractérisée par le fait que** dans le polymère à motifs (i) acrylamide, (ii) halogénure de dialkyldiallylammonium, et (iii) acide carboxylique vinylique, le motif halogénure de dialkyldiallylammonium est le chlorure de diméthyldiallylammonium.

11. Composition selon l'une quelconque des revendications précédentes, **caractérisée par le fait que** dans le polymère à motifs (i) acrylamide, (ii) halogénure de dialkyldiallylammonium, et (iii) acide carboxylique vinylique, le motif acide carboxylique vinylique est choisi parmi l'acide acrylique, l'acide méthacrylique, et l'acide 2-acrylamido-2-méthyl-propane sulfonique.

12. Composition selon la revendication 11, **caractérisée par** le fait le motif (iii) est l'acide acrylique.

13. Composition selon l'une quelconque des revendications précédentes, **caractérisée par le fait que** le polymère à motifs (i) acrylamide, (ii) halogénure de dialkyldiallylammonium, et (iii) acide carboxylique vinylique est le terpolymère acrylamide / chlorure de diméthyldiallylammonium / acide acrylique.

14. Composition selon l'une quelconque des revendications précédentes, **caractérisée par le fait que** le ou les polymères à motifs (i) acrylamide, (ii) halogénure de dialkyldiallylammonium, et (iii) acide carboxylique vinylique sont présents dans la proportion de 0,1 à 10% en poids par rapport au poids total de la composition.

15. Composition selon la revendication 14, **caractérisée par le fait que** le ou les polymères à motifs (i) acrylamide, (ii) halogénure de dialkyldiallylammonium, et (iii) acide carboxylique vinylique sont présents dans la proportion de 0,5 à 5% en poids par rapport au poids total de la composition.

16. Composition selon la revendication 15, **caractérisée par le fait que** le ou les polymères à motifs (i) acrylamide, (ii) halogénure de dialkyldiallylammonium, et (iii) acide carboxylique vinylique sont présents dans la proportion de 1 à 3% en poids par rapport au poids total de la composition.

17. Composition selon l'une quelconque des revendications précédentes, **caractérisée par le fait que** le ou les polymères associatifs sont présents dans la proportion de 0,1 à 10% en poids par rapport au poids total de la composition.

18. Composition selon la revendication 17, **caractérisée par le fait que** le ou les polymères associatifs sont présents dans la proportion de 0,5 à 5% en poids par rapport au poids total de la composition.

19. Composition selon la revendication 18, **caractérisée par le fait que** le ou les polymères associatifs sont présents dans la proportion de 1 à 3% en poids par rapport au poids total de la composition.

20. Composition selon l'une quelconque des revendications précédentes, **caractérisée par le fait qu'**elle contient en outre au moins un polymère cationique ou amphotère différent des polymères associatifs et des polymères à motifs (i) acrylamide, (ii)halogénure de dialkyldiallylammonium, et (iii) acide carboxylique vinylique décrits à l'une quelconque des revendications précédentes.

21. Composition selon la revendication 20, **caractérisée par le fait que** le polymère cationique ou amphotère est choisi parmi :
- 1/ les homopolymères de chlorure de diméthyldiallylammonium;
- 2/ les polymères au motifs récurrents de formules (W) ou (U) suivantes : 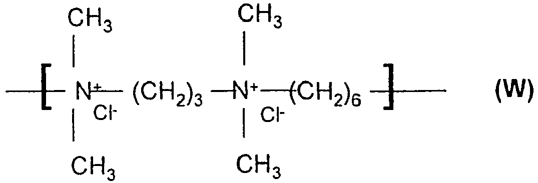 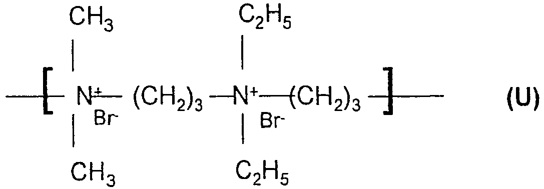
- 3/ les polymères aux motifs de formule (IX) suivante : 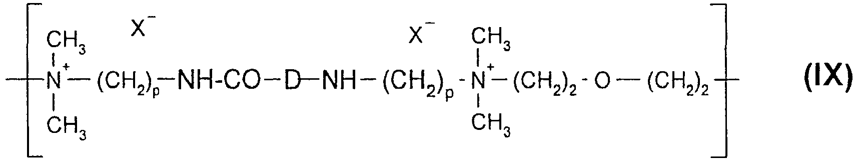 pour laquelle, p est égal à 3, et,
a) D désigne la valeur zéro, X désigne un atome de chlore,
b) D représente un groupement -(CH₂)₄-CO-, X désigne un atome de chlore,
c) D représente un groupement -(CH₂)₇-CO-, X désigne un atome de chlore,
d) un " Block Copolymer " formé de motifs correspondant aux polymères décrits aux alinéas a) et b);
- 4/ les copolymères d'acide acrylique et de chlorure de diméthyldiallylammonium.

22. Composition selon l'une des revendications 20 ou 21, **caractérisée par le fait que** le ou lesdits polymères cationiques ou amphotères sont présents dans la proportion de 0,01 à 10% en poids par rapport au poids total de la composition.

23. Composition selon l'une quelconque des revendications précédentes, **caractérisée par le fait qu'**elle contient au moins un agent tensioactif choisi parmi les tensioactifs anioniques, cationiques, non ioniques ou amphotères ou zwittérioniques dans la proportion d'au moins 0,01% en poids par rapport au poids total de la composition.

24. Composition selon la revendication 23, **caractérisée par le fait que** l'agent tensio-actif est non ionique.

25. Composition selon la revendication 6, **caractérisée par le fait qu'**elle possède un pH compris entre 3 et 12.

26. Composition selon la revendication 25, **caractérisée par le fait que** le pH est compris entre 8,5 et 11.

27. Composition selon la revendication 6, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

28. Composition selon la revendication 28, **caractérisée par le fait que** l'agent oxydant est le peroxyde d'hydrogène.

29. Composition selon la revendication 28, **caractérisée par le fait que** l'agent oxydant est une solution d'eau oxygénée dont le titre varie de 1 à 40 volumes.

30. Procédé de teinture des fibres kératiniques, en particulier des fibres kératiniques humaines et plus particulièrement les cheveux, **caractérisé par le fait qu'**il consiste à appliquer sur les fibres au moins une composition colorante comprenant, dans un milieu approprié pour la teinture, au moins un colorant (direct ou d'oxydation), la couleur étant révélée à pH alcalin, neutre ou acide à l'aide d'une composition oxydante contenant au moins un agent oxydant, qui est mélangée juste au moment de l'emploi à la composition colorante ou qui est appliquée séquentiellement sans rinçage intermédiaire, au moins un polymère associatif anionique et au moins un polymère à motifs (i) acrylamide, (ii)halogénure de dialkyldiallylammonium, et (iii) acide carboxylique vinylique défini selon les revendications 9 à 14 étant présents dans la composition colorante ou dans la composition oxydante ou dans chacune desdites compositions ; ledit polymère associatif anionique étant choisi parmi les polymères suivants :
- **(II)** ceux comportant au moins un motif hydrophile de type acide carboxylique insaturé oléfinique et au moins un motif hydrophobe de type ester d'alkyl (C₁₀-C₃₀) d'acide carboxylique insaturé, choisi parmi l'acide acrylique, l'acide méthacrylique et l'acide éthacrylique,
- **(V)** les copolymères comportant parmi leurs monomères un acide carboxylique à insaturation α,β-monoéthylénique et un ester d'acide carboxylique à insaturation α,β-monoéthylénique et d'un alcool gras oxyalkyléné.

31. Procédé selon la revendication 30, **caractérisé par le fait que** le ou les polymères associatifs et le ou les polymères à motifs (i) acrylamide, (ii) halogénure de dialkyldiallylammonium, et (iii) acide carboxylique vinylique sont présents dans la composition colorante.

32. Procédé selon les revendications 30 ou 31, **caractérisé par le fait qu'**il consiste à appliquer sur les fibres kératiniques sèches ou humides, la composition prête à l'emploi, réalisée extemporanément au moment de l'emploi à partir des compositions colorante et oxydante, à la laisser agir pendant un temps de pause variant de 1 à 60 minutes environ, et de préférence de 5 à 45 minutes, à rincer les fibres, puis éventuellement à les laver au shampooing, puis à les rincer à nouveau, et à les sécher.

33. Dispositif à plusieurs compartiments ou « Kit » pour la teinture des fibres kératiniques, en particulier des fibres kératiniques humaines et plus particulièrement les cheveux, **caractérisé par le fait qu'**il comporte au moins deux compartiments, dont l'un d'entre eux renferme une composition colorante comprenant, dans un milieu approprié pour la teinture, au moins un colorant (direct ou d'oxydation), et un autre une composition oxydante comprenant, dans un milieu approprié pour la teinture, un agent oxydant, au moins un polymère associatif anionique et au moins un polymère à motifs (i) acrylamide, (ii)halogénure de dialkyldiallylammonium, et (iii) acide carboxylique vinylique défini selon les revendications 9 à 14 étant présents dans la composition colorante ou dans la composition oxydante ou dans chacune desdites compositions ; ledit polymère associatif anionique étant choisi parmi les polymères suivants :
- **(II)** ceux comportant au moins un motif hydrophile de type acide carboxylique insaturé oléfinique et au moins un motif hydrophobe de type ester d'alkyl (C₁₀-C₃₀) d'acide carboxylique insaturé, choisi parmi l'acide acrylique, l'acide méthacrylique et l'acide éthacrylique,
- **(V)** les copolymères comportant parmi leurs monomères un acide carboxylique à insaturation α,β-monoéthylénique et un ester d'acide carboxylique à insaturation α,β-monoéthylénique et d'un alcool gras oxyalkyléné.

## Patentansprüche

1. Zusammensetzung zum Färben von Keratinfasern, insbesondere menschlichen Keratinfasern und spezieller Haaren, die in einem für die Färbung geeigneten Medium mindestens einen Oxidationsfarbstoff und mindestens ein anionisches Assoziativpolymer umfasst und **dadurch gekennzeichnet ist, dass** sie außerdem mindestens ein Polymer mit (i) Acrylamid-, (ii) Dialkyldiallylammoniumhalogenid- und (iii) Vinylcarbonsäure-Einheiten umfasst, wobei das anionische Assoziativpolymer aus den folgenden Polymeren ausgewählt ist:
- (II) denjenigen, die mindestens eine hydrophile Einheit vom Typ olefinische ungesättigte Carbonsäure und mindestens eine hydrophobe Einheit vom Typ (C₁₀-C₃₀)-Alkylester einer ungesättigten Carbonsäure, ausgewählt aus Acrylsäure, Methacrylsäure und Ethacrylsäure, umfassen,
- (V) Copolymeren, die unter ihren Monomeren eine Carbonsäure mit α,β-monoethylenischer Ungesättigtheit und einen Ester einer Carbonsäure mit α,β-monoethylenischer Ungesättigtheit und eines oxyalkylenierten Fettalkohols umfassen.

2. Zusammensetzung nach Anspruch 1, **dadurch gekennzeichnet, dass** es sich bei dem Oxidationsfarbstoff bzw. den Oxidationsfarbstoffen um Oxidationsbasen und/oder Kuppler handelt.

3. Zusammensetzung nach Anspruch 2, **dadurch gekennzeichnet, dass** die Oxidationsbasen aus ortho- und para-Phenylendiaminen, Doppelbasen, ortho- und para-Aminophenolen, heterocyclischen Basen und Additionssalzen dieser Verbindungen mit einer Säure ausgewählt sind und in Konzentrationen von 0,0005 bis 12 Gew.-%, bezogen auf das Gesamtgewicht der Zusammensetzung, vorliegen.

4. Zusammensetzung nach Anspruch 2, **dadurch gekennzeichnet, dass** die Kuppler unter meta-Aminophenolen, meta-Phenylendiaminen, meta-Diphenolen, Naphtholen, heterocyclischen Kupplern und Additionssalzen dieser Verbindungen mit einer Säure ausgewählt sind und in Konzentrationen von 0,0001 bis 10 Gew.-%, bezogen auf das Gesamtgewicht der Zusammensetzung, vorliegen.

5. Zusammensetzung nach einem der Ansprüche 3 oder 4, **dadurch gekennzeichnet, dass** die Säureadditionssalze der Oxidationsfarbstöffe (Basen und Kuppler) aus Hydrochloriden, Hydrobromiden, Sulfaten, Tartraten, Lactaten und Acetaten ausgewählt sind.

6. Zusammensetzung nach Anspruch 2, **dadurch gekennzeichnet, dass** sie außerdem mindestens ein Oxidationsmittel enthält.

7. Zusammensetzung nach Anspruch 6, **dadurch gekennzeichnet, dass** sie außerdem mindestens einen Direktfarbstoff in einem Anteil von 0,001 bis 20 Ges.-%, bezogen auf das Gesamtgewicht der Zusammensetzung, umfasst.

8. Zusammensetzung nach einem der vorhergehenden Ansprüche, **dadurch gekennzeichnet, dass** in dem Polymer mit (i) Acrylamid-, (ii) Dialkyldiallylammoniumhalogenid- und (iii) Vinylcarbonsäure-Einheiten die Acrylamid-Einheit aus den Einheiten der folgenden Struktur (I) ausgewählt ist: 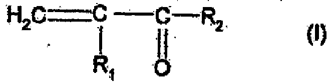 worin
- R₁ für H oder CH₃ steht,
- R₂ aus einem Amino-, Dimethylamino-, tert.-Butylamino-, Dodecylamino- oder -NH-CH₂OH-Rest ausgewählt ist.

9. Zusammensetzung nach Anspruch 9, **dadurch gekennzeichnet, dass** in der Formel (I) R₁ für Wasserstoff steht und R₂ für einen Aminorest steht.

10. Zusammensetzung nach einem der vorhergehenden Ansprüche, **dadurch gekennzeichnet, dass** es sich in dem Polymer mit (i) Acrylamid-, (ii) Dialkyldiallylammoniumhalogenid- und (iii) Vinylcarbonsäure-Einheiten bei der Dialkyldiallylammoniumhalogenid-Einheit um Dimethyldiallylammoniumchlorid handelt.

11. Zusammensetzung nach einem der vorhergehenden Ansprüche, **dadurch gekennzeichnet, dass** in dem Polymer mit (i) Acrylamid-, (ii) Dialkyldiallylammoniumhalogenid- und (iii) vinylcarbonsäure-Einheiten die Vinylcarbonsäure-Einheit aus Acrylsäure, Methacrylsäure und 2-Acrylamido-2-methylpropansulfonsäure ausgewählt ist.

12. Zusammensetzung nach Anspruch 11, **dadurch gekennzeichnet, dass** es sich bei der Einheit (iii) um Acrylsäure handelt.

13. Zusammensetzung nach einem der vorhergehenden Ansprüche, **dadurch gekennzeichnet, dass** es sich bei dem Polymer mit (i) Acrylamid-, (ii) Dialkyldiallylammoniumhalogenid- und (iii) Vinylcarbonsäure-Einheiten um Acrylamid/Dimethyldiallylammoniumchlorid/Acrylsäure-Terpolymer handelt.

14. Zusammensetzung nach einem der vorhergehenden Ansprüche, **dadurch gekennzeichnet, dass** das oder die Polymere mit (i) Acrylamid-, (ii) Dialkyldiallylammoniumhalogenid- und (iii) Vinylcarbonsäure-Einheiten in einem Anteil von 0,1 bis 10 Gew.-%, bezogen auf das Gesamtgewicht der Zusammensetzung, vorliegen.

15. Zusammensetzung nach Anspruch 14, **dadurch gekennzeichnet, dass** das oder die Polymere mit (i) Acrylamid-, (ii) Dialkyldiallylammoniumhalogenid- und (iii) Vinylcarbonsäure-Einheiten in einem Anteil von 0,5 bis 5 Gew.-%, bezogen auf das Gesamtgewicht der Zusammensetzung, vorliegen.

16. Zusammensetzung nach Anspruch 15, **dadurch gekennzeichnet, dass** das oder die Polymere mit (i) Acrylamid-, (ii) Dialkyldiallylammoniumhalogenid- und (iii) Vinylcarbonsäure-Einheiten in einem Anteil von 1 bis 3 Gew.-%, bezogen auf das Gesamtgewicht der Zusammensetzung, vorliegen.

17. Zusammensetzung nach einem der vorhergehenden Ansprüche, **dadurch gekennzeichnet, dass** das oder die Assoziativpolymere in einem Anteil von 0,1 bis 10 Gew.-%, bezogen auf das Gesamtgewicht der Zusammensetzung, vorliegen.

18. Zusammensetzung nach Anspruch 17, **dadurch gekennzeichnet, dass** das oder die Assoziativpolymere in einem Anteil von 0,5 bis 5 Gew.-%, bezogen auf das Gesamtgewicht der Zusammensetzung, vorliegen.

19. Zusammensetzung nach Anspruch 18, **dadurch gekennzeichnet, dass** das oder die Assoziativpolymere in einem Anteil von 1 bis 3 Gew.-%, bezogen auf das Gesamtgewicht der Zusammensetzung, vorliegen.

20. Zusammensetzung nach einem der vorhergehenden Ansprüche, **dadurch gekennzeichnet, dass** sie außerdem mindestens ein kationisches oder amphoteres Polymer, das von den Assoziativpolymeren und den Polymeren mit (i) Acrylamid-, (ii) Dialkyldiallylammoniumhalogenid- und (iii) Vinylcarbonsäure-Einheiten gemäß einem der vorhergehenden Ansprüche verschieden ist, umfasst.

21. Zusammensetzung nach Anspruch 20, **dadurch gekennzeichnet, dass** das kationische oder amphotere Polymer aus:
- 1/ Dimethyldiallylammoniumchlorid-Hömopolymeren;
- 2/ Polymeren mit Wiederholungseinheiten der folgenden Formeln (W) oder (U): 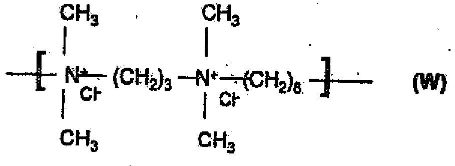 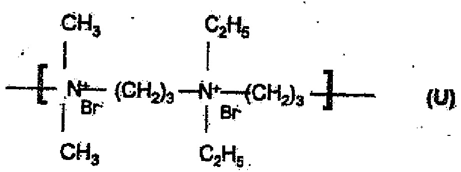
- 3/ Polymeren mit Einheiten der folgenden Formel (IX) : 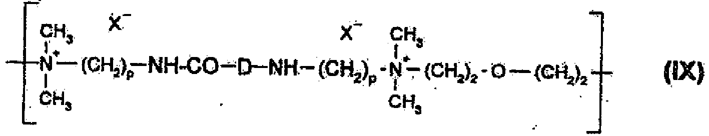 wobei p gleich 3 ist und
a) D den Wert null bedeutet, X ein Chloratom bedeutet,
b) D für eine -(CH₂)₄-CO-Gruppe steht, X ein Chloratom bedeutet,
c) D für eine -(CH₂)₇-CO-Gruppe steht, X ein Chloratom bedeutet,
d) ein "Blockcopolymer" aus Einheiten, die den in den Absätzen a) und b) beschriebenen Polymeren entsprechen;
- 4/ Copolymeren von Acrylsäure und Dimethyldiallylammoniumchlorid
ausgewählt ist.

22. Zusammensetzung nach einem der Ansprüche 20 oder 21, **dadurch gekennzeichnet, dass** das oder die kationischen oder amphoteren Polymere in einem Anteil von 0,01 bis 10 Gew.-%, bezogen auf das Gesamtgewicht der Zusammensetzung, vorliegen.

23. Zusammensetzung nach einem der vorhergehenden Ansprüche, **dadurch gekennzeichnet, dass** sie außerdem mindestens ein Tensid, das aus anionischen, kationischen, nichtionischen oder amphoteren oder zwitterionischen Tensiden ausgewählt ist, in einem Anteil von mindestens 0,01 Gew.-%, bezogen auf das Gesamtgewicht der Zusammensetzung, umfasst.

24. Zusammensetzung nach Anspruch 23, **dadurch gekennzeichnet, dass** das Tensid nichtionisch ist.

25. Zusammensetzung nach Anspruch 6, **dadurch gekennzeichnet, dass** sie einen pH-Wert zwischen 3 und 12 aufweiset.

26. Zusammensetzung nach Anspruch 25, **dadurch gekennzeichnet, dass** sie einen pH-Wert zwischen 8,5 und 11 aufweist.

27. Zusammensetzung nach Anspruch 6, **dadurch gekennzeichnet, dass** das Oxidationsmittel aus Wasserstoffperoxid, Harnstoffperoxid, Alkalimetallbromaten oder -ferricyaniden, Persalzen und Redoxenzymen, gegebenenfalls in Gegenwart ihres jeweiligen Donors oder Cofaktors, ausgewählt ist.

28. Zusammensetzung nach Anspruch 28, **dadurch gekennzeichnet, dass** es sich bei dem Oxidationsmittel um Wasserstoffperoxid handelt.

29. Zusammensetzung nach Anspruch 28, **dadurch gekennzeichnet, dass** es sich bei dem Oxidationsmittel um eine wässrige Wasserstoffperoxidlösung mit einem Titer von 1 bis 40 Volumina handelt.

30. Verfahren zum Färben von Keratinfasern, insbesondere menschlichen Keratinfasern und spezieller Haaren, **dadurch gekennzeichnet, dass** man auf die Fasern mindestens eine Färbezusammensetzung aufbringt, die in einem für die Färbung geeigneten Medium mindestens einen Farbstoff (Direkt- oder Oxidationsfarbstoff), wobei die Farbe bei alkalischem, neutralem oder saurem pH-Wert mit Hilfe einer Oxidationszusammensetzung, die mindestens ein Oxidationsmittel enthält und zum Zeitpunkt des Einsatzes der Färbezusammensetzung beigemischt oder sequentiell ohne Zwischenspülung aufgebracht wird, entwickelt wird, mindestens ein anionisches Assoziativpolymer und mindestens ein Polymer mit (i) Acrylamid-, (ii) Dialkyldiallylammoniumhalogenid- und (iii) Vinylcarbonsäure-Einheiten gemäß den Ansprüchen 9 bis 14, die in der Färbezusammensetzung oder in der Oxidationszusammensetzung oder in jeder der Zusammensetzungen vorliegen, umfasst; wobei das anionische Assoziativpolymer aus den folgenden Polymeren ausgewählt ist:
- (II) denjenigen, die mindestens eine hydrophile Einheit vom Typ olefinische ungesättigte Carbonsäure und mindestens eine hydrophobe Einheit vom Typ (C₁₀-C₃₀)-Alkylester einer ungesättigen Carbonsäure, ausgewählt aus Acrylsäure, Methacrylsäure und Ethacrylsäure, umfassen,
- (V) Copolymeren, die unter ihren Monomeren eine Carbonsäure mit α,β-monoethylenischer Ungesättigtheit und einen Ester einer Carbonsäure mit α,β-monoethylenischer Ungesättigtheit und eines oxyalkylenierten Fettalkohols umfassen.

31. Verfahren nach Anspruch 30, **dadurch gekennzeichnet, dass** das oder die Assoziativpolymere und das oder die Polymere mit (i) Acrylamid-, (ii) Dialkyldiallylammoniumhalogenid- und (iii) Vinylcarbonsäure-Einheiten in der Färbezusammensetzung vorliegen.

32. Verfahren nach den Ansprüchen 30 oder 31, **dadurch gekennzeichnet, dass** man auf die trockenen oder feuchten Keratinfasern die unmittelbar vor dem Zeitpunkt des Einsatzes aus der Färbezusammensetzung und der Oxidationszusammensetzung hergestellte gebrauchsfertige Zusammensetzung aufbringt, über eine Wartezeit von ungefähr 1 bis 60 Minuten und vorzugsweise 5 bis 45 Minuten einwirken lässt, die Fasern spült, dann gegebenenfalls mit Shampoo wäscht, dann erneut spült und trocknet.

33. Vorrichtung mit mehreren Kompartimenten oder "Kit" zum Färben von Keratinfasern, insbesondere menschlichen Keratinfasern und spezieller Haaren, **dadurch gekennzeichnet, dass** sie mindestens zwei Kompartimente umfasst, von denen das eine eine Färbezusammensetzung, die in einem für die Färbung geeigneten Medium mindestens einen Farbstoff (Direkt- oder Oxidationsfarbstoff) umfasst, enthält und ein anderes eine Oxidationszusammensetzung, die in einem für die Färbung geeigneten Medium ein Oxidationsmittel, mindestens ein anionisches Assoziativpolymer und mindestens ein Polymer mit (i) Acrylamid-, (ii) Dialkyldiallylammoniumhalogenid- und (iii) Vinylcarbonsäure-Einheiten gemäß den Ansprüchen 9 bis 14, die in der Färbezusammensetzung oder in der OxidationsZusammensetzung oder in jeder der Zusammensetzungen vorliegen, umfasst; wobei das anionische Assoziativpolymer aus den folgenden Polymeren ausgewählt ist:
- (II) denjenigen, die mindestens eine hydrophile Einheit vom Typ olefinische ungesättigte Carbonsäure und mindestens eine hydrophobe Einheit vom Typ (C₁₀-C₃₀) -Alkylester einer ungesättigten Carbonsäure, ausgewählt aus Acrylsäure, Methacrylsäure und Ethacrylsäure, umfassen,
- (V) Copolymeren, die unter ihren Monomeren eine Carbonsäure mit α,β-monoethylenischer Ungesättigtheit und einen Ester einer Carbonsäure mit α,β-monoethylenischer Ungesättigtheit und eines oxyalkylenierten Fettalkohols umfassen.

## Claims

1. Composition for the dyeing of keratin fibres, in particular of human keratin fibres and more particularly the hair, comprising, in a medium that is suitable for dyeing, at least one oxidation dye and at least one anionic associative polymer, and which is **characterized in that** it also contains at least one polymer containing (i) acrylamide, (ii) dialkyldiallylammonium halide and (iii) vinylcarboxylic acid units;
said anionic associative polymer being chosen from the following polymers:
- **(II)** those comprising at least one hydrophilic unit of unsaturated olefinic carboxylic acid type, and at least one hydrophobic unit of (C₁₀-C₃₀) alkyl ester of unsaturated carboxylic acid type chosen from acrylic acid, methacrylic acid and ethacrylic acid,
- **(V)** copolymers comprising among their monomers an α,β-monoethylenically unsaturated carboxylic acid and an ester of an α,β-monoethylenically unsaturated carboxylic acid and of an oxyalkylenated fatty alcohol.

2. Composition according to Claim 1, **characterized in that** the oxidation dye(s) is(are) chosen from oxidation bases and/or couplers.

3. Composition according to Claim 2, **characterized in that** the oxidation bases are chosen from ortho- or para-phenylenediamines, double bases, ortho- or para-aminophenols and heterocyclic bases, and also the addition salts of these compounds with an acid, and are present in concentrations ranging from 0.0005% to 12% by weight relative to the total weight of the composition.

4. Composition according to Claim 2, **characterized in that** the couplers are chosen from meta-aminophenols, meta-phenylenediamines, meta-diphenols, naphthols and heterocyclic couplers, and the addition salts of these compounds with an acid, and are present in concentrations ranging from 0.0001% to 10% by weight relative to the total weight of the composition.

5. Composition according to either of Claims 3 and 4, **characterized in that** the addition salts with an acid of the oxidation dyes (bases and couplers) are chosen from the hydrochlorides, hydrobromides, sulfates, tartrates, lactates and acetates.

6. Composition according to Claim 2, **characterized in that** it also contains at least one oxidizing agent.

7. Composition according to Claim 6, **characterized in that** it also contains at least one direct dye in a proportion of from 0.001% to 20% by weight relative to the total weight of the composition.

8. Composition according to any one of the preceding claims, **characterized in that**, in the polymer containing (i) acrylamide, (ii) dialkyldiallylammonium halide and (iii) vinylcarboxylic acid units, the acrylamide unit is chosen from the units of structure (I) below: 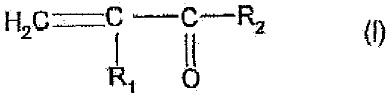 in which:
- R₁ denotes H or CH₃,
- R₂ is chosen from an amino, dimethylamino, tert-butylamino, dodecylamino or -NH-CH₂OH radical.

9. Composition according to Claim 9, **characterized in that**, in formula (I), R₁ is hydrogen and R₂ is an amino radical.

10. Composition according to any one of the preceding claims, **characterized in that**, in the polymer containing (i) acrylamide, (ii) dialkyldiallylammonium halide and (iii) vinylcarboxylic acid units, the dialkyldiallylammonium halide unit is dimethyldiallylammonium chloride.

11. Composition according to any one of the preceding claims, **characterized in that**, in the polymer containing (i) acrylamide, (ii) dialkyldiallylammonium halide and (iii) vinylcarboxylic acid units, the vinylcarboxylic acid unit is chosen from acrylic acid, methacrylic acid and 2-acrylamido-2-methylpropanesulfonic acid.

12. Composition according to Claim 11, **characterized in that** the unit (iii) is acrylic acid.

13. Composition according to any one of the preceding claims, **characterized in that** the polymer containing (i) acrylamide, (ii) dialkyldiallylammonium halide and (iii) vinylcarboxylic acid units is the acrylamide/dimethyldiallylammonium chloride/acrylic acid terpolymer.

14. Composition according to any one of the preceding claims, **characterized in that** the polymer(s) containing (i) acrylamide, (ii) dialkyldiallylammonium halide and (iii) vinylcarboxylic acid units is(are) present in a proportion of from 0.1% to 10% by weight relative to the total weight of the composition.

15. Composition according to Claim 14, **characterized in that** the polymer(s) containing (i) acrylamide, (ii) dialkyldiallylammonium halide and (iii) vinylcarboxylic acid units is(are) present in a proportion of from 0.5% to 5% by weight relative to the total weight of the composition.

16. Composition according to Claim 15, **characterized in that** the polymer(s) containing (i) acrylamide, (ii) dialkyldiallylammonium halide and (iii) vinylcarboxylic acid units is(are) present in a proportion of from 1% to 3% by weight relative to the total weight of the composition.

17. Composition according to any one of the preceding claims, **characterized in that** the associative polymer(s) is(are) present in a proportion of from 0.1% to 10% by weight relative to the total weight of the composition.

18. Composition according to Claim 17, **characterized in that** the associative polymer(s) is(are) present in a proportion of from 0.5% to 5% by weight relative to the total weight of the composition.

19. Composition according to Claim 18, **characterized in that** the associative polymer(s) is(are) present in a proportion of from 1% to 3% by weight relative to the total weight of the composition.

20. Composition according to any one of the preceding claims, **characterized in that** it also contains at least one cationic or amphoteric polymer other than the associative polymers and than the polymers containing (i) acrylamide, (ii) dialkyldiallylammonium halide and (iii) vinylcarboxylic acid units described in any one of the preceding claims.

21. Composition according to Claim 20, **characterized in that** the cationic or amphoteric polymer is chosen from:
- 1/ dimethyldiallylammonium chloride homopolymers;
- 2/ polymers containing the repeating units of formula (W) or (U) below: 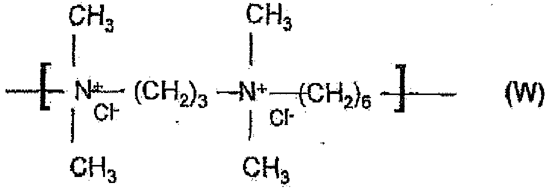 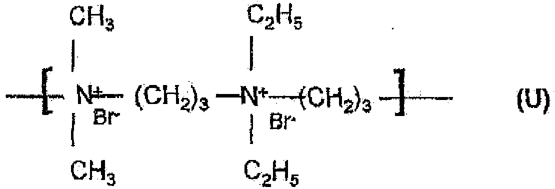
- 3/ polymers containing the units of formula (IX) below: 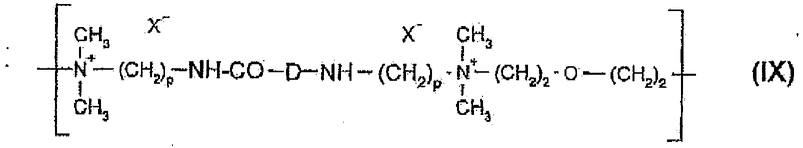 for which p is equal to 3, and
a) D denotes the value zero, X denotes a chlorine atom,
b) D represents a -(CH₂)₄-CO- group, X denotes a chlorine atom,
c) D represents a -(CH₂)₇-CO- group, X denotes a chlorine atom,
d) a "Block Copolymer" formed from units corresponding to the polymers described in paragraphs a) and b);
- 4/ copolymers of acrylic acid and of dimethyldiallylammonium chloride.

22. Composition according to either of Claims 20 and 21, **characterized in that** the said cationic or amphoteric polymer(s) is(are) present in a proportion of from 0.01% to 10% by weight relative to the total weight of the composition.

23. Composition according to any one of the preceding claims, **characterized in that** it contains at least one surfactant chosen from anionic, cationic, nonionic, amphoteric and zwitterionic surfactants, in a proportion of at least 0.01% by weight relative to the total weight of the composition,

24. Composition according to Claim 23, **characterized in that** the surfactant is nonionic.

25. Composition according to Claim 6, **characterized in that** it has a pH of between 3 and 12.

26. Composition according to Claim 25, **characterized in that** the pH is between 8.5 and 11.

27. Composition according to Claim 6, **characterized in that** the oxidizing agent is chosen from hydrogen peroxide, urea peroxide, alkali metal bromates or ferricyanides, persalts, and redox enzymes optionally with the respective donor or cofactor thereof.

28. Composition according to Claim 28, **characterized in that** the oxidizing agent is hydrogen peroxide.

29. Composition according to Claim 28, **characterized in that** the oxidizing agent is an aqueous hydrogen peroxide solution whose titre ranges from 1 to 40 volumes.

30. Process for the dyeing of keratin fibres, in particular of human keratin fibres and more particularly the hair, **characterized in that** it consists in applying to the fibres at least one dye composition comprising, in a medium that is suitable for dyeing, at least one dye (direct dye or oxidation dye), the colour being developed at alkaline, neutral or acidic pH using an oxidizing composition containing at least one oxidizing agent, which is mixed with the dye composition just at the time of use or which is applied sequentially without intermediate rinsing, at least one anionic associative polymer and at least one polymer containing (i) acrylamide, (ii) dialkyldiallylammonium halide and (iii) vinylcarboxylic acid units defined according to Claims 9 to 14 being present in the dye composition or in the oxidizing composition or in each of the said compositions;
- **(II)** those comprising at least one hydrophilic unit of unsaturated olefinic carboxylic acid type, and at least one hydrophobic unit of (C₁₀-C₃₀)alkyl ester of unsaturated carboxylic acid type chosen from acrylic acid, methacrylic acid and ethacrylic acid,
- (V) copolymers comprising among their monomers an α,β-monoethylenically unsaturated carboxylic acid and an ester of an α,β-monoethylenically unsaturated carboxylic acid and of an oxyalkylenated fatty alcohol.

31. Process according to Claim 30, **characterized in that** the associative polymer(s) and the polymer(s) containing (i) acrylamide, (ii) dialkyldiallylammonium halide and (iii) vinylcarboxylic acid units are present in the dye composition.

32. Process according to Claim 30 or 31, **characterized in that** it consists in applying to wet or dry keratin fibres the ready-to-use composition prepared extemporaneously at the time of use from the dye composition and oxidizing composition, in leaving the composition to act for an exposure time ranging from 1 to 60 minutes approximately, and preferably from 5 to 45 minutes, in rinsing the fibres and then in optionally washing them with shampoo, then rinsing them again and drying them.

33. Multi-compartment device or "kit" for the dyeing of keratin fibres, in particular of human keratin fibres and more particularly the hair, **characterized in that** it comprises at least two compartments, one of which contains a dye composition comprising, in a medium that is suitable for dyeing, at least one dye (direct dye or oxidation dye), and another containing an oxidizing composition comprising, in a medium that is suitable for dyeing, an oxidizing agent, at least one anionic associative polymer and at least one polymer containing (i) acrylamide, (ii) dialkyldiallylammonium halide and (iii) vinylcarboxylic acid units defined according to Claims 9 to 14 being present in the dye composition or in the oxidizing composition or in each of the said compositions;
- **(II)** those comprising at least one hydrophilic unit of unsaturated olefinic carboxylic acid type, and at least one hydrophobic unit of (C₁₀-C₃₀) alkyl ester of unsaturated carboxylic acid type chosen from acrylic acid, methacrylic acid and ethacrylic acid,
- **(V)** copolymers comprising among their monomers an α,β-monoethylenically unsaturated carboxylic acid and an ester of an α,β-monoethylenically unsaturated carboxylic acid and of an oxyalkylenated fatty alcohol.
